# EUROPÄISCHE PATENTSCHRIFT

(11) **EP 2 771 044 B1**
(45) Veröffentlichungstag und Bekanntmachung des Hinweises auf die Patenterteilung: **27.07.2016**
(21) Anmeldenummer: 12784528.7
(22) Anmeldetag: 24.10.2012
(51) Int. Cl.: A61M 1/10

(54) **KÜNSTLICHES HERZ**
ARTIFICIAL HEART
COEUR ARTIFICIEL

(30) Priorität: 25.10.2011 DE 102011054768
(43) Veröffentlichungstag der Anmeldung: 03.09.2014
(73) Patentinhaber: Kargakis, Stavros, 4731 Raeren (BE)
(72) Erfinder: Kargakis, Stavros, 4731 Raeren (BE)
(74) Vertreter: Bauer, Dirk
(86) Internationale Anmeldenummer: PCT/EP2012/071011
(87) Internationale Veröffentlichungsnummer: WO 2013/060701

(56) Entgegenhaltungen:
- DE-A1- 2 449 320
- US-A- 4 969 864
- US-A- 5 300 111
- US-A- 5 314 469
- US-B1- 6 478 820

## Beschreibung

Die Erfindung betriff ein künstliches Herz, insbesondere zur Nachbildung eines menschlichen Herzens, umfassend einen Energiespeicher, eine Antriebseinrichtung sowie zwei Förderkammern mit jeweils einer Eintrittsöffnung sowie einer Austrittsöffnung, wobei jede Förderkammer mindestens von einer flexiblen und einer starren Wandung begrenzt ist und ein Volumen jeder Förderkammer durch Verformung der flexiblen Wandung mittels der Antriebseinrichtung veränderbar ist, indem die Wandung ausgehend von einer Saugstellung, in der das Volumen der jeweiligen Förderkammer maximal ist, mittels der Antriebseinrichtung in eine Druckstellung überführbar ist, in der das Volumen der Speicherkammer minimal ist, wobei die Eintrittsöffnung und die Austrittsöffnung einer jeden Förderkammer jeweils ein Rückschlagventil aufweisen und die Rückschlagventile wechselseitig in eine geöffnete und eine geschlossene Stellung bringbar sind, wodurch ein gerichteter Blutstrom von der jeweiligen Eintrittsöffnung zu der jeweiligen Austrittsöffnung erzeugbar ist, wobei die Antriebseinrichtung zwischen den Förderkammern angeordnet ist.

### Stand der Technik

Künstliche Herzen der eingangs beschriebenen Art sind bereits aus dem Stand der Technik bekannt, wenngleich sie heutzutage nach wie vor technisch nicht vollständig ausgereift und entsprechend selten im praktischen Einsatz sind.

Insbesondere die europäische Patentschrift EP 0 971 756 B1 stellt ein künstliches Herz vor, das über zwei V-förmig angeordnete künstliche Herzkammern beziehungsweise Ventrikel verfügt, die jeweils von einem Motorpumpenaggregat angetrieben werden, wobei das aus den Herzkammern gebildete V umgedreht ist, also gewissermaßen auf dem Kopf steht. Die beiden künstlichen Herzkammern werden jeweils durch eine Membraneinheit, die über eine der beiden jeweilig zugehörigen Motorpumpenaggregate verformt wird, betrieben, wobei ein Volumen der jeweiligen Herzkammer im Verlauf des Betriebs des Herzens zwischen einem Maximum und einen Minimum wechselt. Die Membraneinheiten der Herzkammern werden mittels eines hydraulischen Drucks angesteuert, wobei die Motorpumpenaggregate ein in einem jeweiligen Druckraum befindliches Fluid, dass auf einer der jeweiligen Herzkammer abgewandten Seite der jeweiligen Membraneinheit anliegt, mit Druck beaufschlag wird. Die Membraneinheiten können auf diese Weise flächig durch Aufbringen eines gleichmäßigen Drucks verformt werden.

Mit Ausnahme eines Energiespeichers ist die gesamte Steuerungselektronik sowie -mechanik des künstlichen Herzens innerhalb eines festen Gehäuses eingeschlossen, wobei dieses Gehäuse über insgesamt vier Kanäle mit dem Blutkreislauf eines Menschen verbunden werden kann. Jeweils zwei dieser vier Kanäle sind jeweils mit einer der beiden Herzkammern verbunden und simulieren im Fall der rechten Herzkammer die Hohlvene(n) und die Lungenarterie beziehungsweise im Fall der linken Herzkammer die Lungenvene(n) und die Aorta.

Als nachteilig bei der in der EP 0 971 756 B1 gezeigten Vorrichtung ist der relativ große Platzbedarf sowie das verhältnismäßig hohe Gewicht des gesamten Herzens im Vergleich zu einem natürlichen Herz zu beurteilen. Außerdem sind die "Anschlüsse" zur Verbindung des Herzens mit dem Blutkreislauf des Patienten vornehmlich an den Aufbau des Herzens angepasst, unterscheiden sich in ihrer Lage deutlich von einem natürlichen Herz, so dass ein Anschluss der Blutgefäße bei einer Implantation dieses künstlichen Herzens umständlich ausfällt.

Ein weiteres Beispiel für ein künstliches Herz, bei dem die beiden Herzkammern in einer V-Form zueinander angeordnet sind, zeigt die EP 0 079 373 B1, wobei eine Orientierung des V's in diesem Fall richtig herum ist, das V also nicht auf dem Kopf steht. Von der prinzipiellen Funktionsweise ist das hier gezeigte künstliche Herz der des Herzens aus der vorgenannten Schrift durchaus ähnlich, wobei eine Anordnung der Pumpenaggregate unterschiedlich ausfällt. Dabei handelt es sich abermals um zwei Pumpenaggregate, wobei jeweils eines von beiden für den Betrieb einer der Herzkammern zuständig ist. Die Herzkammern werden auf einer Seite durch eine flexible Membran begrenzt, die durch den hydraulischen Druck eines Fluids verformbar sind, der durch die Pumpenaggregate erzeugt wird. Die Pumpenaggregate sind dabei unterhalb der Herzkammern angeordnet, während sie im Fall der zuvor genannten EP 0 971 756 B1 neben selbigen platziert wurden.

Auch diese Vorrichtung weist vornehmlich das Problem auf, dass der Platzbedarf denjenigen eines natürlichen Herzens deutlich übersteigt und somit in seiner Funktionalität zwar ausreichend sein könnte, in der praktischen Anwendung jedoch zu Problemen führen würde.

Ein weiteres künstliches Herz ist in der EP 0 324 669 B1 offenbart, wobei ein Unterschied des hier gezeigten Herzens im Vergleich zu den zuvor genannten Schriften in erster Linie in der Anordnung der Antriebseinrichtungen liegt, die hier pro Herzkammer jeweils durch ein elektromechanisches Organ in Verbindung mit einer jeweilig zugehörigen Mikropumpe gebildet werden. Hinsichtlich des Platzbedarfs und der Form des künstlichen Herzens ist die hier gezeigte Anordnung gegenüber den zuvor genannten Schriften noch etwas verschlechtert, da die Antriebseinrichtungen jeweils in etwa senkrecht auf einer Mittelebene der V-förmig angeordneten Herzkammern stehen und ausgehend von den Herzkammern in den Körper des Patienten ragen. Ferner ist das in dieser Schrift gezeigte Herz dahingehend als nachteilig zu beurteilen, als dass es auf eine externe, das heißt, außerhalb eines das Herz einschließenden Hüllkörpers angeordnete Ausgleichskammer zurückgreift, die einen zusätzlichen Platzbedarf innerhalb des Körpers des Patienten sowie einen zusätzlichen Verbindungsaufwand mit dem Herz verursacht.

Ferner zeigt die DE 23 37 497 B2 ein künstliches Herz, bei dem zusätzlich zu den Herzkammern auch die Vorhöfe explizit mit abgebildet sind und auch mittels eigener Membranen betätigt werden. Dies erfolgt durch eine wechselseitige Schaltung einer Herzkammer und des jeweilig zugeordneten Vorhofes, wobei eine Membran, die dem Vorhof zugeordnet ist, bewirkt, dass der Blutstrom aus dem Vorhof in die Herzkammer weitergeleitet wird, während die Membran, die der Herzkammer zugeordnet ist, den Blutstom in die Aorta beziehungsweise in die Lungenarterie pumpt, je nachdem, um welche der Herzkammern (links oder rechts) es sich handelt. Die Erzeugung eines hydraulischen Drucks auf ein Übertragungsfluid, welches wiederum auf die Membranen wirkt, wird mittels zweier Antriebseinrichtungen erzeugt, wobei jeweils eine Antriebseinrichtung einer Herzseite zugeordnet ist, wobei ein Pumpenkolben einer jeweiligen Antriebseinrichtung durch Magnetismus bewegt wird. Das gezeigte Herz ist neben dem hohen Gewicht und dem hohen Platzbedarf vor allem deswegen von Nachteil, weil bei der gezeigten Anordnung stets Totwassergebiete im Blutstrom auftreten, die letztendlich zur Gerinnung des Blutes aus dem Blutstrom beitragen, so dass das Herz in der Praxis nicht einsetzbar ist.

Aus der DE 24 49 320 geht ferner ein künstliches Herz hervor, bei dem eine Antriebseinrichtung zwischen den Herzkammern angeordnet ist. Dies hat den Vorteil, dass mittels einer einzigen Antriebseinrichtung beide Herzkammer angetrieben werden können, wobei mittels der mittigen Antriebseinrichtung verschiebliche Wandteile der Herzkammern in eine der Antriebseinrichtung abgewandte Richtung "nach außen" gedrückt werden können und dabei das Volumen der jeweiligen Herzkammer reduzieren. Eine Rückstellung der beweglichen Wandteile in die ursprüngliche Position erfolgt mittels Rückstellfedern. Die Nachteile bei diesem künstlichen Herz sind gleich denen aus der vorgenannten DE 23 37 497 B2.

### Aufgabe

Der vorliegenden Erfindung liegt die Aufgabe zugrunde, ein künstliches Herz hervorzubringen, welches sich insbesondere sowohl durch naturnahe äußere Abmessungen sowie durch eine Masse als auch durch eine Funktionsweise auszeichnet, die mit der eines natürlichen Herzens vergleichbar und folglich in die Perikardhöhle implantierbar ist.

### Lösung

Die Erfindung wird durch die Merkmale des unabhängigen Anspruchs 1 definiert.

Die Aufgabe wird ausgehend von einem künstlichen Herz der eingangs beschriebenen Art erfindungsgemäß dadurch gelöst, dass die Förderkammern lang gestreckt und bananenförmig ausgeformt sind, wobei eine Mittellinie einer jeweiligen Förderkammer gekrümmt ist und vorzugsweise einen Krümmungsradius zwischen 10 cm und 40 cm, weiter vorzugsweise zwischen 15 cm und 30 cm, aufweist und ein senkrecht zu der Mittellinie einer jeweiligen Förderkammer geführter Querschnitt durch die Förderkammer in der Saugstellung eine Form einer Ellipse aufweist, wobei ein Verhältnis zwischen einer großen Halbachse und einer kleinen Halbachse der Ellipse zwischen 1,0 und 2,0, vorzugsweise zwischen 1,1 und 1,5, beträgt.

Die beschriebene Form der Förderkammern kann umgangssprachlich auch als "Bananenform", "Nierenform" oder "Sichelzellform" bezeichnet werden, wobei die

Förderkammern vorzugsweise eine Länge von mindestens 10 cm aufweisen, während die große Halbachse mindestens 1,0 cm und die kleine Halbachse mindestens 0,5 cm betragen. Die Länge der Förderkammern entspricht der Länge einer beide Enden der jeweiligen. Förderkammer verbindenden Geraden, das heißt die Länge einer Förderkammer ist nicht gleich einer Länge ihrer gekrümmten Mittellinie. Stattdessen beschreibt die Mittellinie einer Förderkammer einen Kreisbogen, wobei die Länge der Förderkammer der Länge einer die Enden des Kreisbogens verbindenden Sehne entspricht. Die Dimensionen Länge, große Halbachse und kleine Halbachse sind dabei stets so aufeinander abgestimmt, dass die jeweilige Förderkammer ein Volumen zwischen 60 cm³ und 120 cm³, vorzugsweise ein Volumen von 80 cm³, aufweist. Die Mittellinie einer Förderkammer ist als Verbindungslinie der Schwerpunkte sämtlicher Querschnitte durch die Förderkammer auffassbar.

Versuche haben zeigen können, dass die beschriebene Form der Förderkammern für den Bluttransport besonders vorteilhaft ist, wobei sich die flexiblen Wandungen der Förderkammern in dem Sinne besonders gut an die starre Wandung anlegen, dass keine Totwassergebiete entstehen. Die Gefahr der Blutgerinnung ist daher bei dem erfindungsgemäßen künstlichen Herzen nicht gegeben.

In einer vorteilhaften Ausgestaltung des erfindungsgemäßen Herzens ist ein Innenraum einer jeweiligen Förderkammer von eine elastische Hülle begrenzt, wobei die elastische Hülle in einem von der Antriebseinrichtung abgewandten Stützbereich, vorzugsweise über eine gesamte Länge der Förderkammer, durch Zusammenwirken mit einem im Querschnitt U-förmigen starren Stützkörper die starre Wandung der Förderkammer ausbildet und außerhalb des Stützbereichs die elastische Hülle die flexible Wandung ausbildet, wobei vorzugsweise in einem Querschnitt durch die elastische Hülle senkrecht zur Mittellinie der jeweiligen Förderkammer betrachtet eine Hälfte eines Umfangs der elastischen Hülle mit dem U-förmigen Stützkörper zusammenwirkt. Das heißt, dass die starre Wandung und die flexible Wandung nicht jeweils für sich allein von einem bestimmten Körper gebildet sind, sondern gewissermaßen die ganze Förderkammer von einer flexiblen Wandung gebildet ist, wobei diese allein durch das Zusammenwirken mit dem starren Stützkörper die Funktion einer starren Wandung erhält. Überall dort, wo die elastische Hülle nicht mittels des Stützkörpers gestützt ist, ist sie flexibel und elastisch verformbar und kann sich an die starre, mittels des Stützkörpers versteifte Wandung anlegen.

In einer besonders vorteilhaften Ausgestaltung dieser Kombination von starrem Stützkörper und elastischer Hülle sind die elastische Hülle und der Stützkörper mittels eines Formschlusses in Kraft übertragender Weise miteinander verbunden, wobei vorzugsweise der Stützkörper über dessen Länge mindestens eine hinterschnittene Nut aufweist, in die eine korrespondierende Feder der elastischen Hülle eingreift. Vorteilhafterweise weist der Stützkörper mehrere dieser Nuten und analog die elastische Hülle mehrere entsprechende Federn auf, damit die Verbindung zwischen beiden Teilen besonders zuverlässig ist. Alternativ oder zusätzlich zu der beschriebenen Variante ist auch eine kraftschlüssige Verbindung zwischen den beiden Teilen denkbar, wobei insbesondere eine flächige Verklebung des Stützkörpers mit der Hülle vorteilhaft sein kann. Besonders vorteilhaft kann eine Kombination eines Forrrischlusses und eines Kraftschlusses zwischen dem Stützkörper und der Hülle sein.

Die Förderkammern, die die Herzkammer eines natürlichen Herzens nachzubilden versuchen, sind im Sinne der vorliegenden Erfindung vorzugsweise nebeneinander angeordnet, wobei im Stand der Technik eine V-förmige beziehungsweise eine umgekehrte V-förmige Anordnung der beiden Herzkammern weit verbreitet ist, also zusätzlich zur beabstandeten Anordnung beider Förderkammern außerdem eine gegenseitige Verdrehung derselben vorgesehen ist. Für das erfindungsgemäße künstliche Herz wird es hingegen als besonders vorteilhaft angesehen, wenn die Förderkammern parallel zueinander angeordnet sind, wobei unter einer parallelen Anordnung hierbei zu verstehen ist, dass zwei jeweilig einer Förderkammer zugehörige Ebenen parallel zueinander angeordnet sind, wobei diese Ebenen tangential an den jeweiligen Förderkammern anliegen und jeweils durch einen Hochpunkt der zugehörigen Förderkammer verlaufen, wobei dieser Hochpunkt in der starren Wandung der jeweiligen Förderkammer angeordnet ist und in seiner Lage dadurch definiert ist, dass für den Fall, in dem die flexible Wandung in ihrer Saugstellung ist, ein senkrecht zu der starren Wandung gemessener Abstand derselben zu der flexiblen Wandung maximal ist. Im Vergleich zur V-förmigen Anordnung ist der Raum zwischen den Förderkammern bei einer derartigen parallelen Anordnung größer und daher deutlich besser nutzbar. Ferner ergeben sich durch die parallele Anordnung der Förderkammern Vorteile für den Antrieb der flexiblen Wandungen derselben, wie später gesondert erläutert werden wird.

Zum Schutz des gesamten künstlichen Herzens sollte möglichst ein Hüllkörper vorgesehen werden, der beide Förderkammern sowie die Antriebseinrichtung vollständig umschließt. Ein solcher Hüllkörper kann zum Beispiel aus einem starren Material, beispielsweise einem beschichteten Kunststoff, gefertigt sein, so dass mechanische Einwirkungen, die von außen auf den Patienten wirken, abgefangen werden können und somit das Herz vor einer Beschädigung geschützt ist. Neben der Platzierung der Förderkammern und der Antriebseinrichtung kann ferner die Anordnung des Energiespeichers, aus dem letztlich die Antriebseinrichtung mit Energie gespeist wird, innerhalb des Hüllkörpers vorteilhaft sein, so dass eine gesonderte Verkabelung des Herzens mit einem externen Bauteil vollständig entfallen kann. In Anbetracht der aktuellen Speichertechniken zur Speicherung elektrischer Energie bietet eine Anordnung des Energiespeichers außerhalb des Hüllkörpers jedoch den großen Vorteil, dass ein Wechsel des Energiespeichers deutlich einfacher durchgeführt werden kann, zum Beispiel indem dieser gut erreichbar nahe an der Hautoberfläche des Patienten platziert wird. Für den Fall, dass größere Speicherkapazitäten realisiert werden können, wäre jedoch eine integrale Ausführung sowohl des Energiespeichers, das heißt, eine Anordnung des Energiespeichers im Inneren des Hüllkörpers, als auch der übrigen Bauteile des Herzens besonders vorteilhaft.

Besonders vorteilhaft ist ein solches erfindungsgemäßes künstliches Herz, bei dem bei einer der beiden Förderkammern eine Anordnung der zugehörigen Austrittsöffnung einer natürlichen Anordnung einer Lungenarterie einer rechten Herzkammer des menschlichen Herzens entspricht. Vorteilhafterweise sollte es sich dabei analog zur rechten Herzkammer beim natürlichen Herzen um die rechte Förderkammer handeln. Die naturnahe Lage der Austrittsöffnung ist insbesondere im Zuge einer Implantation des künstlichen Herzens von Vorteil, da eine aufwendige Ankoppelung desselben an die natürliche Lungenarterie entfällt. Dies spart Zeit während der Implantation und verhilft dem Patienten somit zu einem geringeren Behandlungsrisiko sowie dem behandelnden Arzt zu einer effizienteren Arbeitsweise.

Dieser Vorteil ist hinsichtlich der linken Förderkammer analog auf die Anordnung der Aorta übertragbar. Bei einer der beiden Förderkammern sollte entsprechend die Austrittsöffnung der natürlichen Lage der Aorta des natürlichen Herzens entsprechen.

Zur weiteren Annäherung der grundsätzlichen Anatomie des künstlichen Herzens an das natürliche Herz sollte ferner bei einer der beiden Förderkammern auf einer der Förderkammer abgewandten Seite der zugehörigen Eintrittsöffnung ein Anschlussraum angeordnet sein, wobei vorzugsweise zwei Leitungen zur Leitung eines Blutstromes in diesen Anschlussraum münden. Der Anschlussraum ist über die Eintrittsöffnung mit der jeweiligen Förderkammer verbunden, wobei in der Eintrittsöffnung das eingangs beschriebene Rückschlagventil angeordnet ist. Er dient somit lediglich der Zusammenführung der separaten Blutlaufbahnen. Das Rückschlagventil ist vorteilhafterweise unter Verwendung eines Clip-Systems lösbar in der jeweiligen Eintrittsöffnung beziehungsweise Austrittsöffnung zwischen Anschlussraum und Förderkammer angeordnet, so dass es besonders einfach austauschbar ist. Durch eine Anordnung der beschriebenen Förderkammer, die den beschriebenen Anschlussraum aufweist, auf eine solche Weise, dass sie als "Ersatz" der rechten Herzkammer verwendet wird, ist entsprechend ein Anschluss des künstlichen Herzens an die Hohlvenen besonders einfach möglich. Optimalerweise ist die Förderkammer, die den erläuterten Anschlussraum mit zwei in ihm mündenden Leitungen aufweist, gleichzeitig dieselbe Förderkammer, deren Lage der zugehörigen Austrittsöffnung der Lage der Lungenarterie beim natürlichen Herzen nachempfunden ist. Auf diese Weise ist diese Förderkammer optimal der Geometrie der rechten Herzkammer des natürlichen Herzens nachgebildet, so dass es besonders einfach ist, das Herz unter Beibehaltung der natürlichen Gefäße bei einem Patienten zu implantieren. Analog ist es besonders von Vorteil, wenn bei einer der beiden Förderkammern auf einer der jeweiligen Förderkammer abgewandten Seite der zugehörigen Eintrittsöffnung ein Anschlussraum angeordnet ist, in den vorzugsweise vier Leitungen zur Leitung eines Blutstromes in diesen Anschlussraum münden. Anknüpfend an vorstehende Erläuterung hinsichtlich des Einsatzes einer Förderkammer des erfindungsgemäßen künstlichen Herzens als "Ersatz" der rechten Herzkammer des natürlichen Herzens ist entsprechend die Förderkammer, die einen Anschlussraum mit vier in ihn mündenden Leitungen vorsieht, besonders gut für den Ersatz der linken Herzkammer des natürlichen Herzens geeignet, da diese vier Leitungen die vier Lungenvenen des natürlichen Herzens abbilden können. Analog ist diese Förderkammer vorteilhafterweise eben diejenige Förderkammer, bei der die Austrittsöffnung gemäß vorstehender Erläuterung der Anordnung der Aorta bei einem natürlichen Herz nachempfunden ist.

Unabhängig von der Lage der Anschlüsse beziehungsweise der Ein- und Austrittsöffnungen der Förderkammern ist insbesondere ein solches erfindungsgemäßes künstliches Herz auf besonders vorteilhafte Weise betreibbar, bei dem mindestens eine flexible Wandung mindestens einer Förderkammer mittels mindestens einer Übertragungseinrichtung, vorzugsweise mittels einer Mehrzahl von Übertragungseinrichtungen, bewegt wird, wobei die Übertragungseinrichtung von der Antriebseinrichtung angetrieben wird. Unter der Übertragungseinrichtung sind hier sämtliche Einrichtungen zu verstehen, mittels deren eine von der Antriebseinrichtung erzeugte Kraft auf die flexible Wandung übertragbar ist.

Als eine besonders sinnvolle Ausführung sei hier die Ausbildung der Übertragungseinrichtung als Kolben-Zylinder-Einheit genannt. Diese Kolben-Zylinder-Einheit kann von der Antriebseinrichtung angetrieben werden, indem diese mittels einer Pumpe einen Druck beziehungsweise Antriebsdruck innerhalb des Zylinders der Zylinder-Kolben-Einheit aufbaut. Dieser Druck wirkt ausgehend von einer Anfangsstellung der Kolben-Zylinder-Einheit sowohl auf Wandungen des Zylinders und ebenso auf eine Unterseite des Kolbens, der daraufhin aus dem Zylinder herausgedrückt wird. Dieser Kolben ist schließlich mit der flexiblen Wandung der Förderkammer verbunden, so dass die Bewegung des Kolbens gleichermaßen zu einer Bewegung beziehungsweise Verformung der flexiblen Wandung führt. Diese kann auf diese Weise von der Saugstellung in die Druckstellung überführt werden, wobei die Druckstellung der flexiblen Wandung mit einer Endstellung der Kolben-Zylinder-Einheit korrespondiert, das heißt, dass der Kolben der Kolben-Zylinder-Einheit in dieser Endstellung maximal aus dem Zylinder heraus bewegt ist. Durch den Einsatz einer derartigen Übertragungseinrichtung kann das Volumen der zugehörigen Förderkammer zyklisch zwischen einem Maximum und einem Minimum verändert werden, so dass letztendlich fortwährend Blut aus der Förderkammer verdrängt beziehungsweise neu angesaugt wird und auf diese Weise schließlich ein pulsierender Blutstrom erzeugbar ist.

Alternativ zu einer solchen Anordnung ist gleichermaßen eine rein mechanische Antriebsweise denkbar, bei der die Übertragungseinrichtung von einer Nocken-Stößel-Einheit gebildet wird. Dabei wird mindestens ein Nocken von der Antriebseinrichtung angetrieben, wobei der Nocken mit mindestens einem Stößel verbunden ist, der durch eine Rotation des Nockens eine Hubbewegung ausführt. Analog zum vorstehend beschriebenen Kolben sollte dieser Stößel schließlich mit der flexiblen Wandung einer Förderkammer verbunden sein, so dass diese durch den Stößel verformbar ist.

Die Verwendung einer Nocken-Stößel-Einheit kann vor allem dann besonders vorteilhaft sein, wenn der Nocken derart ausgebildet ist, dass einer eine Vielzahl von Bergen und Tälern aufweist, so dass ein zugehöriger Stößel während einer Umdrehung des Nockens eine Vielzahl von Hüben ausführt. Auf diese Weise kann das erfindungsgemäße künstliche Herz besonders energiesparend eingesetzt werden, da bereits mit einer geringen Drehzahl des Nockens eine Vielzahl von Pumpzyklen beziehungsweise "Herzschlägen" ausführbar und somit ein pulsierender Blutstrom erzeugbar ist.

Ferner bietet die Verwendung einer Nocken-Stößel-Einheit den besonderen Vorteil, dass ein Nocken mehrere, insbesondere zwei, Stößel gleichzeitig antreiben kann. Durch die Anordnung der Antriebseinrichtung zwischen den Förderkammern können auf diese Weise mittels einer Übertragungseinrichtung gleich beide Förderkammern betrieben werden.

Wie bereits erwähnt, sollte das künstliche Herz dabei optimalerweise mit einer Vielzahl von Übertragungseinrichtungen ausgestattet sein, wobei eine Vielzahl von Übertragungseinrichtungen pro Förderkammer beziehungsweise pro flexibler Wandung vorgesehen werden sollte. Dies ist vor allem deswegen von Vorteil, da die flexiblen Wandungen einer jeweiligen Förderkammer bei einer flächenmäßig begrenzten Beaufschlagung mit einer Kraft gegebenenfalls nur lokal verformt wird und weiter von einer solchen Krafteinleitungsstelle entfernt liegende Stellen der flexiblen Wandung unverformt bleiben und somit nicht zur Verdrängung des Blutes aus der Förderkammer beitragen. Insbesondere ist ein Stillstand des Blutes unbedingt zu vermeiden, da es, wie bereits vorstehend erläutert, bei einer nicht kontinuierlichen Strömung des Blutes zur Gerinnung desselben kommen kann, die eine direkte gesundheitliche Gefahr für den Patienten darstellen würde. Durch eine über die flexible Wandung verteilte Krafteinleitung mittels einer Mehrzahl von Übertragungseinrichtungen kann dies ausgeschlossen werden, so dass keine Teilbereiche innerhalb der jeweiligen Förderkammer verbleiben, in denen das Blut nicht weiter gefördert wird. An den konkreten vorteilhaften Ausführungen der Übertragungseinrichtung in Form einer Kolben-Zylinder-Einheit oder einer Nocken-Stößel-Einheit bedeutet dies, dass eine Vielzahl von Kolben beziehungsweise Stößeln jeweils möglichst homogen über die flexiblen Wandungen beider Förderkammern verteilt angreifen sollte, so dass die Wandungen sich möglichst gleichmäßig verformen.

Das Problem der möglichst gleichmäßigen Krafteinleitung in die flexiblen Wandungen der Förderkammern lässt sich abseits der erläuterten Übertragungseinrichtungen besonders einfach mittels einer Beaufschlagung der flexiblen Wandungen mittels eines hydraulischen Drucks lösen. Das künstliche Herz sollte dabei entsprechend so ausgeführt sein, dass mindestens eine flexible Wandung mindestens einer Förderkammer mittels eines hydraulischen Drucks bewegbar ist, wobei die Wandung vorzugsweise auf einer der Förderkammer abgewandten Seite mit einem Übertragungsfluid beaufschlagbar ist, das mittels der Antriebseinrichtung mit einem Druck beaufschlagbar ist, wobei die Antriebseinrichtung eine Pumpe aufweist. Das Übertragungsfluid sollte bei dieser Anordnung in Druckräumen angeordnet sein, die jeweils auf der der jeweiligen Förderkammer abgewandten Seite der flexiblen Wandung angeordnet sind und mit der Pumpe der Antriebseinrichtung verbunden sind, so dass das Übertragungsfluid mit einem Druck beaufschlagt werden kann. Vorteilhafterweise sollte eine einzige Pumpe dazu genutzt werden, um das Übertragungsfluid in beiden Druckräumen gleichzeitig anzusteuern, so dass eine simultane Überführung der flexiblen Wandungen beider Förderkammern von der Saugstellung in die Druckstellung besonders einfach möglich ist. Ferner ist durch einen gleichzeitigen Druckabfall eine gleichzeitige Rücküberführung der flexiblen Wandungen von der Druckstellung in die Saugstellung besonders einfach möglich.

Vorteilhafterweise ist es von Vorteil, wenn neben der eigentlichen Antriebseinrichtung ferner eine zweite Antriebseinrichtung vorhanden ist, die im Falle eines Defekts der ersten Antriebseinrichtung die Funktion des künstlichen Herzens zumindest für einen gewissen Zeitraum aufrecht erhalten kann, so dass die Gelegenheit besteht, die erste Antriebseinrichtung auszutauschen oder zu reparieren.

Mittels einer Verformung der flexiblen Wandungen der Förderkammern über hydrostatischen Druck wird stets eine gleichmäßige Verformung der mit dem Übertragungsfluid in Kontakt stehenden Stellen der flexiblen Wandungen erreicht. Die Problematik lokaler Verformungen entfällt entsprechend.

Optimalerweise sollten die flexiblen Wandungen dabei über ihre gesamte Fläche mit dem Übertragungsfluid in Kontakt stehen und entsprechend über ihre gesamte Fläche mit dem hydraulischen Druck beaufschlagbar sein. Auf diese Weise wird die Bildung von Totwassergebieten in den Förderkammern besonders effektiv vermieden.

Hinsichtlich einer Ausführung der Rückschlagventile ist insbesondere die Verwendung eines Klappelements vorteilhaft, wobei dieses Klappelement vorzugsweise über eine Mehrzahl von Klappsegmenten verfügt, mittels denen bei einer Strömung des Blutstroms in eine Schließrichtung des Klappelements verschlossen ist und umgekehrt bei einer Strömung des Blutstroms in eine Öffnungsrichtung das Klappelement freigegeben ist. Die einzelnen Klappsegmente eines jeweiligen Klappelements sind dabei so ausgebildet, dass sie gleichmäßig verteilt umlaufend an einer Wandung eines jeweilig zu steuernden Öffnungsquerschnitts der zugehörigen Leitung angeordnet sind und ausgehend von einer zu einer Strömungsrichtung senkrechten Orientierung, in der sie sich überlappen und einen Flüssigkeitsstrom unterbinden, in eine Öffnungsstellung "klappbar" sind, in der der Flüssigkeitsstrom freigegeben ist. Die Klappensegmente sind dabei so ausgeführt, dass sie nicht in eine Richtung, in der der Flüssigkeitsstrom unterbunden werden soll, durchschlagen können und diesen somit nicht unfreiwillig freigeben.

Abschließend ist ein solches künstliches Herz besonders vorteilhaft, bei dem mindestens eine der flexiblen Wandungen einzelne Bereiche aufweist, die mittels der Antriebseinrichtung im Zuge einer Überführung der gesamten Wandung von der Saugstellung in die Druckstellung zeitversetzt verformbar sind. Eine solche zeitversetzte Steuerung der Verformung einer jeweiligen flexiblen Wandung kann insbesondere unter Verwendung einer Übertragungseinrichtung der vorstehend beschriebenen Art realisiert werden, indem beispielsweise an unterschiedlichen Stellen der jeweiligen flexiblen Wandung angeordnete Kolben oder Stößel zeitversetzt ausgelöst werden und sich auf diese Weise bestimmte Bereiche der flexiblen Wandung früher auf die starre Wandung zu bewegen und bereits Blut verdrängen, bevor dies in solchen Bereichen der Fall ist, in denen eine jeweilige Übertragungseinrichtung noch nicht aktiviert wurde. Bei einer Ansteuerung der flexiblen Wandungen mittels hydraulischen Drucks ist eine solche Vorgehensweise hingegen nicht möglich.

Die zeitversetzte Verformung beziehungsweise die damit einhergehende zeitversetzte Verdrängung des Blutes aus der jeweiligen Förderkammer kann insbesondere dazu genutzt werden, um den Blutstrom aus der Förderkammer heraus in Richtung einer gewünschten Stelle der Förderkammer zu steuern. Ist beispielsweise die Austrittsöffnung einer Förderkammer in einem oberen Bereich derselben angeordnet, ist es vorteilhaft, das Blut ausgehend von einem unteren Bereich aus der Förderkammer zu verdrängen und auf diese Weise eine Art peristaltische Förderung des Blutes innerhalb der Förderkammer zu erzeugen.

### Ausführungsbeispiele

Das erfindungsgemäße künstliche Herz wird nachstehend anhand von Beispielen, die in den Figuren dargestellt sind, näher erläutert.

Es zeigt:
- Fig. 1:: Einen Schnitt durch ein erfindungsgemäßes künstliches Herz,
- Fig. 2:: Einen Schnitt durch ein weiteres erfindungsgemäßes künstliches Herz,
- Fig. 3:: Einen Schnitt durch ein weiteres erfindungsgemäßes künstliches Herz,
- Fig. 4:: wie Figur 3, jedoch in einem zusätzlichen Querschnitt dargestellt,
- Fig. 5a:: Einen Querschnitt durch eine Förderkammer in deren Saugstellung,
- Fig. 5b:: wie Figur 5a, jedoch in deren Druckstellung und
- Fig. 6a bis 6c:: Details eines Klappelements, das als Rückschlagventil fungiert.

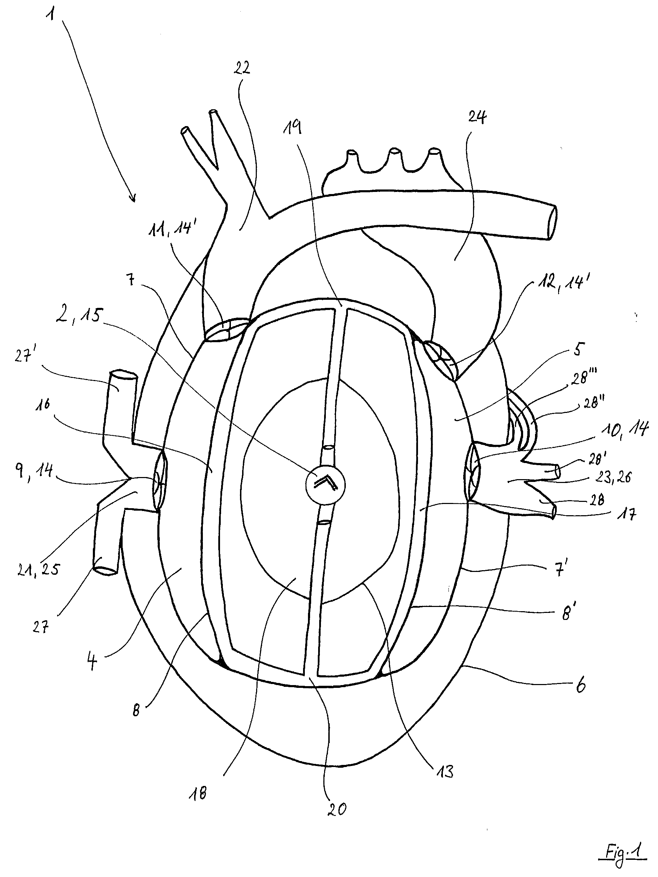

Ein erstes Ausführungsbeispiel, das in Figur 1 dargestellt ist, umfasst ein künstliches Herz 1 mit einer Antriebseinrichtung 2, einem nicht dargestellten Energiespeicher 3 sowie zwei Förderkammern 4, 5, wobei eine rechte Förderkammer 4 auf der linken Seite des gezeigten Herzens 1 und eine linke Förderkammer 5 auf der rechten Seite des gezeigten Herzens 1 angeordnet ist. Das Herz 1 ist mittels eines Hüllkörpers 6 umschlossen und bildet auf diese Weise eine abgeschlossene Einheit. Im Innenraum des Hüllkörpers 6 ist neben den bereits aufgezählten Komponenten ferner eine nicht dargestellte Steuereinheit angeordnet. Diese dient unter anderem einer Regelung einer Schlagfrequenz des Herzens 1. So kann beispielsweise unter körperlicher Belastung die Schlagfrequenz erhöht werden und in einer Ruhephase des Körpers eines Patienten auf einen Ruhepuls beziehungsweise eine Ruhefrequenz heruntergeregelt werden. Der Hüllkörper 6 selbst besteht dabei bevorzugt aus einem biokompatiblen Kunststoffmaterial, das dämpfende Eigenschaften besitzt. So kann beispielsweise ein Silikonelastomer verwendet werden, dessen Oberfläche mit einer biokompatiblen Beschichtung versehen ist.

Die Förderkammern 4, 5 weisen jeweils eine starre Wandung 7, 7' und eine flexible Wandung 8, 8' auf, wobei ein Volumen einer jeden Förderkammer 4, 5 durch eine Verformung der jeweiligen flexiblen Wandung 8, 8' veränderbar ist. In Figur 1 sind beide Wandungen 8, 8' in einer Saugstellung abgebildet, in der das Volumen beider Förderkammern 4, 5 maximal ist. Durch die erwähnte Verformung der flexiblen Wandung 8, 8' in eine Richtung auf die jeweilig zugehörige starre Wandung 7, 7' zu, kann das Volumen der jeweiligen Förderkammer 4, 5 verringert werden, bis die flexible Wandung 8, 8' schließlich eine Druckstellung einnimmt, in der das Volumen der jeweiligen Förderkammer 4, 5 minimal ist. Optimalerweise wird das Volumen der Förderkammern 4, 5 im Zuge einer Überführung der flexiblen Wandungen 8, 8' von ihrer Saugstellung in ihre Druckstellung nahezu auf nullreduziert. Die hier gezeigten Förderkammern 4, 5 weisen jeweils ein Volumen von 80 cm³ auf.

Beide Förderkammern 4, 5 verfügen jeweils über eine Eintrittsöffnung 9, 10 sowie über eine Austrittsöffnung 11, 12, wobei die rechte Förderkammer 4 die Eintrittsöffnung 9 und die Austrittsöffnung 11 und die linke Förderkammer 5 die Eintrittsöffnung 10 und die Austrittsöffnung 12 aufweist. Sämtliche Eintrittsöffnungen 9, 10 und Austrittsöffnungen 11, 12 weisen jeweils ein Rückschlagventil in Form eines Klappelements 14, 14' auf, wobei das Klappelement 14 an den Eintrittsöffnungen 9, 10 derart ausgerichtet ist, dass ein Blutstrom ausschließlich in eine in die jeweiligen Förderkammern 4, 5 hinein gerichtete Richtung leitbar ist und umgekehrt die Klappelemente 14' an den Austrittöffnungen 11, 12 derart ausgerichtet sind, dass der Blutstrom ausschließlich in eine aus den jeweiligen Förderkammern 4, 5 heraus gerichtete Richtung leitbar ist.

Durch eine Veränderung des Volumens der Förderkammern 4, 5 durch die flexiblen Wandungen 8, 8', die mittels der Antriebseinrichtung 2 angetrieben werden, wird in den Förderkammern 4, 5 befindliches Blut bewegt beziehungsweise gefördert. Im Fall der Überführung der flexiblen Wandungen 8, 8' von der Druckstellung in die Saugstellung wird innerhalb der zugehörigen Förderkammern 4, 5 ein Unterdruck erzeugt, der dazu führt, dass außerhalb der Förderkammern 4, 5 befindliches Blut angesaugt wird. Aufgrund der Rückschlagventile in Form der Klappelemente 14 kann jedoch nur Blut durch die jeweiligen Eintrittsöffnungen 9, 10 in die zugehörigen Förderkammern 4, 5 eintreten. Ein in die Förderkammern 4, 5 gerichteter Blutstrom durch die Austrittsöffnungen 11, 12 ist hingegen durch die Klappelemente 14' versperrt. Bei einer anschließenden Überführung der flexiblen Wandungen 8, 8' von der Saugstellung in die Druckstellung wird genau umgekehrt ein Druck in den jeweiligen Förderkammern 4, 5 aufgebaut, der dazu führt, dass das Blut aus dem Inneren der Förderkammern 4, 5 verdrängt wird. Aufgrund der Klappelemente 14 ist ein Entweichen des Blutes jedoch nur durch die Austrittsöffnungen 11, 12 möglich, die Eintrittsöffnungen 9, 10 sind hingegen verschlossen. Durch diesen Ablauf ist schließlich durch das Herz 1 ein gerichteter Blutstrom erzeugbar, der einem natürlichen Herzen ähnlich ist.

Bei dem in Figur 1 gezeigten Ausführungsbeispiel werden die flexiblen Wandungen 8, 8' über einen hydraulischen Druck eines Übertragungsfluids angetrieben. Dieser Druck wird mittels einer Pumpe 15 erzeugt, die Bestandteil der Antriebseinrichtung 2 ist. Diese Pumpe 15 ist zwischen einem Ausgleichsraum 18 und zwei Arbeitsräumen 16, 17 angeordnet, wobei der Arbeitsraum 16 auf einer der Förderkammer 4 abgewandten Seite der flexiblen Wandung 8 und der Arbeitsraum 17 auf einer der Förderkammer 5 abgewandten Seite der flexiblen Wandung 8' angeordnet sind. Beide Arbeitsräume 16, 17 sind miteinander verbunden, so dass das Übertragungsfluid in beiden Arbeitsräumen 16, 17 stets ein gleiches Druckniveau annimmt. Befinden sich die flexiblen Wandungen 8, 8' in der Saugstellung, ist ein Volumen der Arbeitsräume 16, 17 minimal, während ein Volumen des Ausgleichsraums 18, der mittels einer elastischen Wandung 13 umschlossen ist, maximal ist, wobei sämtliche Volumina der Arbeitsräume 16, 17 und des Ausgleichsraums 18 vollständig mit dem Übertragungsfluid gefüllt sind.

Ausgehend von der in der Figur 1 gezeigten Saugstellung der beiden flexiblen Wandungen 8, 8' wird eine Überführung derselben in ihre Druckstellung dadurch erreicht, dass die Pumpe 15 das in dem Ausgleichsraum 18 befindliche Übertragungsfluid in die Arbeitsräume 16, 17 pumpt und auf diese Weise einen Druck auf die Flächen der flexiblen Wandungen 8, 8' aufbaut. Diese verformen sich schließlich unter dem Druck und bewegen sich auf die jeweilig zugehörigen starren Wandungen 7, 7' zu und verdrängen dabei das Blut aus den Förderkammern 4, 5. Um die flexiblen Wandungen 8, 8' vollständig in ihre Druckstellung zu überführen, müssen die Volumina der beiden Arbeitsräume 16, 17 um einen eben solchen Betrag ansteigen, um den die Förderkammern 4, 5 in ihrem Volumen verringert werden. Folglich muss der Ausgleichsraum 18 mindestens ein solches Volumen aufweisen, das mindestens der Summe der Volumina der Förderkammern 4,5 entspricht. Im Zuge der Überführung der flexiblen Wandungen 8, 8' von ihrer Saugstellung in ihre Druckstellung wird folglich der Ausgleichsraum 18 typischerweise annähernd entleert, während die Arbeitsräume 16, 17 analog mit dem Übertragungsfluid gefüllt werden.

Im Zuge der genau umgekehrten Überführung der flexiblen Wandungen 8, 8' von der Druckstellung in die Saugstellung wird das Übertragungsfluid entsprechend aus den Arbeiträumen 16, 17 wieder zurück in den Ausgleichsraum 18 gepumpt. Durch diesen Vorgang wird auf einer den Förderkammern 4, 5 abgewandten Seite der flexiblen Wandungen 8, 8' ein Unterdruck erzeugt, der letztendlich über die Fläche der Wandungen 8, 8' eine Zugkraft bewirkt, die die Wandungen 8, 8' wieder von den jeweilig zugehörigen starren Wandungen 7, 7' weg bewegt. Diese Bewegung der flexiblen Wandungen 8, 8' von der Druckstellung in die Saugstellung wird optimalerweise durch eine Rückstellkraft der Wandungen 8, 8' unterstützt, die aus einer elastischen Verformung derselben herrührt, wobei die Wandungen 8, 8' in ihrer Saugstellung in etwa spannungsfrei und in ihrer Druckstellung maximal ausgelenkt und somit unter Spannung sind. Leitungen 19, 20, über die die Pumpe 15 mit den Arbeitsräumen 16, 17 verbunden ist, sind für beide Bewegungsrichtungen der flexiblen Wandungen 8, 8' mit Ventilen versehen, so dass für die Verdrängung des Blutes aus den Förderkammern 4, 5 das Übertragungsfluid aus dem Ausgleichsraum 18 heraus gepumpt wird und umgekehrt bei Beibehaltung einer Pumprichtung der Pumpe 15 das Übertragungsfluid aus den Arbeitsräumen 16, 17 in den Ausgleichsraum 18 gefördert wird.

Die Eintrittsöffnungen 9, 10 und Austrittsöffnungen 11, 12 der Förderkammern 4, 5 sind analog zu einem natürlichen Herz mit Leitungen 21, 22, 23 ,24 versehen, die an natürliche Blutgefäße eines Menschen angeschlossen werden, so dass einzelne Leitungen 21, 22, 23, 24 mit einzelnen natürlichen Blutgefäßen korrespondieren. Im Fall der rechten Förderkammer 4 bedeutet dies, dass die auf einer der Förderkammer 4 abgewandten Seite der Eintrittsöffnung 9 angeordnete Leitung 21 den Hohlvenen des Menschen entspricht beziehungsweise mit diesen verbunden ist und die auf einer der Förderkammer 4 abgewandten Seite der Austrittsöffnung 11 angeordnete Leitung 22 der Lungenarterie des Menschen entspricht beziehungsweise mit dieser verbunden ist. Analog hierzu korrespondieren die Leitungen 23, 24 der linken Förderkammer 5 mit den Lungenvenen beziehungsweise der Aorta eines Menschen.

Die Leitungen 21, 23, die jeweils mit den Eintrittsöffnungen 9, 10 der beiden Förderkammern 4, 5 verbunden sind, weisen im Unterschied zu den Leitungen 22, 24 jeweils einen Anschlussraum 25, 26 auf, wobei an diese Anschlussräume 25, 26 jeweils eine Mehrzahl von kleinen Leitungen 27, 27', 28, 28', 28", 28"' angeschlossen ist. Dabei sind die Leitungen 27, 27' dem Anschlussraum 25 zugeordnet, der wiederum mit der Eintrittsöffnung 9 und somit mit der rechten Förderkammer 4 korrespondiert und die Leitungen 28, 28', 28", 28'" sind mit dem Anschlussraum 26 verbunden, der mit der Eintrittsöffnung 10 und somit mit der linken Förderkammer 5 korrespondiert. Die Leitungen 27, 27' sind dabei zwei Hohlvenen zugeordnet während die Leitungen 28, 28', 28", 28'" vier Lungenvenen entsprechen. Das Blut aus sämtlichen Leitungen 27, 27', 28, 28', 28", 28'" sammelt sich in den jeweilig zugehörigen Anschlussräumen 25, 26, bevor es durch die zugehörigen Eintrittsöffnungen 9, 10 in die Förderkammern 4, 5 gepumpt beziehungsweise gezogen wird.

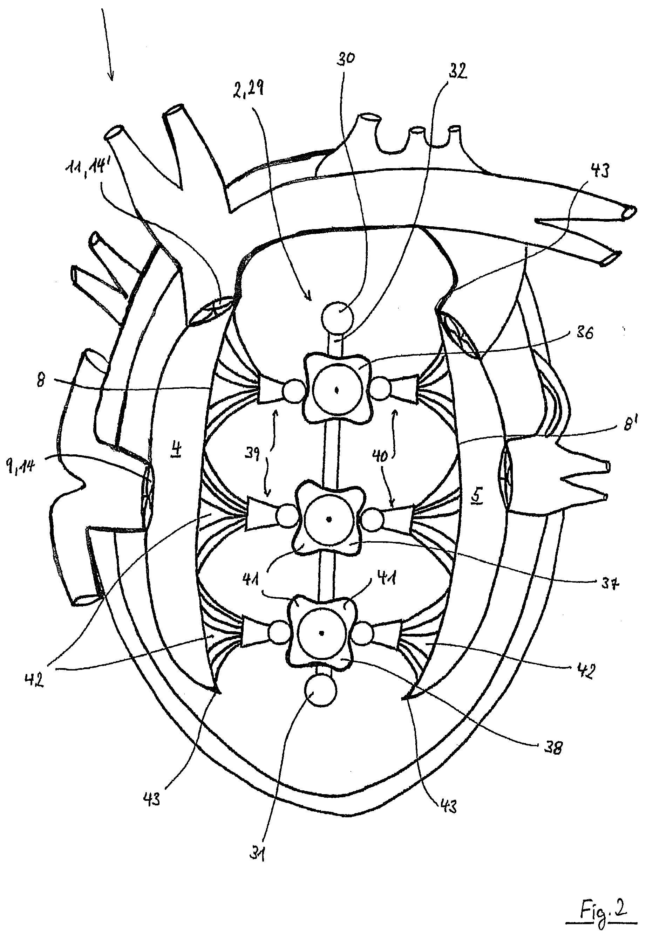

In Figur 2 ist ein weiteres Ausführungsbeispiel abgebildet, wobei die Antriebseinrichtung 2 des dort gezeigten Herzens 1' nicht durch eine Pumpe 15, sondern durch Antriebsmechanik 29 gebildet ist. Entsprechend zeigt das Herz 1' gemäß Figur 2 keine Arbeitsräume 15, 16 und keinen Ausgleichsraum 18.

Die Antriebsmechanik 29 ist stattdessen aus zwei Motoren 30, 31 sowie einer Schneckenwelle 32 gebildet, wobei die Schneckenwelle 32 in nicht dargestellte Zahnräder 33, 34, 35 eingreift, die wiederum jeweils starr mit einem Nocken 36, 37, 38 verbunden sind. Bewirken nun die Motoren 30, 31 eine Rotation der Schneckenwelle 32, versetzt letztere hierdurch die Zahnräder 33, 34, 35 in Rotation, wodurch letztendlich die Nocken 36, 37, 38 ebenfalls beginnen zu rotieren. Auf den Nocken 36, 37, 38 sind jeweils zwei Stößel 39, 40 angeordnet, die durch die Rotation der jeweilig zugehörigen Nocken 36, 37, 38 eine Hubbewegung ausführen. Zu diesem Zweck sind die Nocken 36, 37 ,38 mit jeweils vier Erhebungen 41 versehen, die im Zuge der Rotation der Nocken 36, 37, 38 eine Anhebung der Stößel 39 bewirken. Durch die Anordnung der vier Erhebungen 41 auf jedem der Nocken 36, 37, 38 führen die Stößel 39, 40 insgesamt vier Hubbewegungen pro einer Umdrehung der Nocken 36, 37, 38 aus.

Die Stößel 39, 40 sind mittels einer nicht dargestellten Führungseinrichtung linear geführt, so dass sie sich ausschließlich entlang einer Geraden bewegen können. An einer den Nocken 36, 37, 38 abgewandten Seite sind die Stößel 39, 40 ferner jeweils mit einer Auffächerung 42 versehen, die mit einem dem jeweilig zugehörigen Stößel 39,40 abgewandten Ende mit einer zugehörigen flexiblen Wandung 8, 8' verbunden ist. Auf diese Weise ist eine Kraft übertragende Verbindung zwischen den Stößeln 39 ,40 und den flexiblen Wandungen 8, 8' hergestellt, die die Hubbewegungen der Stößel 39, 40 letztendlich in Hubbewegungen der flexiblen Wandungen 8, 8' umsetzt. Eine vollständige Hubbewegung der Stößel 39, 40 von einer Tiefstellung, in der ein Abstand eines jeweiligen Stößels 39, 40 von einer Rotationsachse des zugehörigen Nockens 36, 37, 38 minimal ist, hin zu einer Hochstellung, in der ein Abstand des jeweiligen Stößels 39, 40 von der Rotationsachse des zugehörigen Nockens maximal ist, und wieder zurück in die Tiefstellung, bewirkt entsprechend eine Überführung der jeweilig zugehörigen flexiblen Wandung 8, 8' von der Saugstellung in die Druckstellung und wieder zurück in die Saugstellung. Die Auffächerungen 42 an den einzelnen Stößeln 39, 40 dienen dabei einer möglichst homogenen Kraftübertragung von den Stößeln 39, 40 auf die flexiblen Wandungen 8, 8', so dass die Gefahr eine lediglich lokalen Verformung der flexiblen Wandungen 8, 8' nicht aufkommt.

Dabei ist zu erläutern, dass eine Rücküberführung der flexiblen Wandungen 8, 8' von der Druckstellung in die Saugstellung an dem in Figur 2 gezeigten Beispiel durch die flexiblen Wandungen 8, 8' selbst bewirkt wird. Diese sind aus einem elastischen Material gefertigt und in einem Randbereich 43 an die zugehörigen starren Wandungen 7, 7' angeschlossen. Die flexiblen Wandungen 8, 8' werden in ihrer Saugstellung in das Herz 1' eingebaut, wobei sie mit einer leichten Vorspannung versehen werden, die bestrebt ist, die jeweilige flexible Wandung 8, 8' "gerade" zu ziehen, das heißt, sie in eine ebene Form zu bringen. Dies wird lediglich durch die Stößel 39, 40, die sich in ihrer Tiefstellung befinden, verhindert, so dass auf die Stößel 39, 40 eine Axialkraft wirkt, die selbige auf die jeweilig zugehörigen Nocken 36, 37, 38 drückt. Werden die Nocken 36, 37, 38 schließlich mittels der Motoren 30, 31 in Rotation versetzt und im Zuge dessen die Stößel 39, 40 von ihrer Tiefstellung in ihre Hochstellung überführt, wird die flexible Wandung 8, 8' zusätzlich zu ihrer Vorspannung elastisch verformt, wodurch sich die beschriebene Rückstellkraft weiter erhöht. Die Motoren 30, 31 wirken dieser Rückstellkraft allerdings entgegen, so dass die Nocken 36, 37, 38 weiter rotieren und die Stößel 39, 40 entgegen der Rückstellkraft anheben. Haben die Stößel 39, 40 schließlich ihre Hochstellung erreicht - befinden sich also die flexiblen Wandungen 8, 8' in ihrer Druckstellung - bewirkt schließlich die Rückstellkraft der flexiblen Wandungen 8, 8', dass die Stößel 39, 40, sobald sie über ihre Hochstellung hinaus sind, nicht "abheben", das heißt, einen Kontakt zu den weiter rotierenden Nocken 36, 37, 38 nicht verlieren, sondern vielmehr Vertiefungen zwischen den Erhebungen 41 der Nocken 36, 37, 38 gedrückt werden. Die von vornherein aufgebrachte Vorspannung bewirkt dabei, dass ein solches Abheben auch in der Tiefstellung der Stößel 39, 40 nicht auftritt.

Besonders vorteilhaft bei dem in Figur 2 gezeigten Ausführungsbeispiel ist die Anordnung zweier Stößel 39, 40 pro Nocken 36, 37, 38, da auf diese Weise beide flexiblen Wandungen 8, 8' gleichzeitig bewegt werden können. Diese Bewegung erfolg in Anlehnung an ein natürliches Herz gleichzeitig, das heißt, dass sich sämtliche Stößel 39, 40 in etwa gleichzeitig in ihrer Hochstellung beziehungsweise ihrer Tiefstellung befinden, die flexiblen Wandungen 8, 8' also entsprechend gleichzeitig in ihrer Saugstellung beziehungsweise ihrer Druckstellung sind.

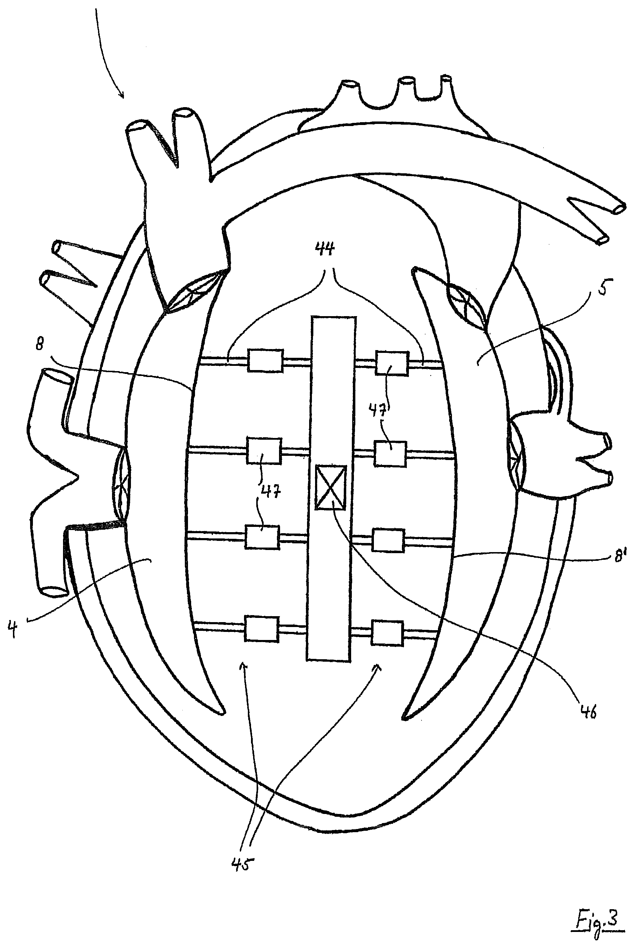

In einem weiteren Ausführungsbeispiel, das in Figur 3 dargestellt ist, wird die Verformung der flexiblen Wandungen 8, 8' der Förderkammern 4, 5 eines Herzens 1" mittels Kolben 44 bewirkt, die jeweils Bestandteil einer Kolben-Zylinder-Einheit 45 sind. Um eine möglichst gleichmäßige Krafteinleitung in die Wandungen 8, 8' zu erreichen wird vorteilhafterweise - wie im gezeigten Beispiel - eine Vielzahl von Kolben 44 verwendet. Auf diese Art und Weise wird lokal begrenzten Verformungen der flexiblen Wandungen 8, 8' vorgebeugt, die gegebenenfalls dazu führen könnten, dass sich einzelne "Dellen" bilden, in denen sich Blut sammelt und daraufhin still steht, was zu einer Gerinnung des Blutes führen könnte.

Die Kolben 44 der Kolben-Zylinder-Einheiten 45 werden mittels einer Pumpe 46, die Bestandteil der Antriebseinrichtung 2 ist, mit einem Druck beaufschlagt, wobei die Pumpe 46 auf ein Übertragungsfluid wirkt, welches dadurch unter Druck gesetzt wird. Dieses Übertragungsfluid ist in Zylindern 47 der Kolben-Zylinder-Einheiten 45 angeordnet, so dass durch eine Anhebung eines Druckniveaus des Übertragungsfluids der Zylinder 47 mit ebendiesem Druckniveau beaufschlagt wird, wobei ebenfalls eine Unterseite eines jeweiligen in einem Zylinder 47 angeordneten Kolbens 44 durch diesen Druck von einer Tiefstellung in eine Hochstellung überführt wird. Die Kolben-Zylinder-Einheit 45 erfüllt demnach genau die Funktion der Nocken 36, 37, 38 und Stößel 39, 40 des Ausführungsbeispiels gemäß Figur 2. Die übrigen Funktionsweisen sind als identisch zu betrachten. Bei dem Übertragungsfluid handelt es sich um ein körperverträgliches Fluid, dass im Fall einer Beschädigung des künstlichen Herzens 1" nicht geeignet ist, die Gesundheit des Patienten negativ zu beeinflussen. Zwar ist das Herz 1" grundsätzlich dicht ausgeführt, so dass selbst bei internen Undichtigkeiten kein Fluidaustausch zwischen dem Körper des Patienten und dem Herz 1" stattfinden kann, jedoch könnte im Zuge eines Unfalls dennoch eine derart gravierende Beschädigung des Herzens 1" herbeigeführt werden, dass das Übertragungsfluid austritt.

Die Verwendung von Kolben-Zylinder-Einheiten 45 bietet dabei den Vorteil, dass diese jeweils individuell ansteuerbar sind und auf diese Weise besonders einfach eine zeitversetzte Hubbewegung der einzelnen Kolben 44 bewirkt werden kann. Insbesondere ist es möglich, dass die beiden flexiblen Wandungen 8, 8' unterschiedlich gesteuert werden, während diese sich unter Verwendung des Herzens 1' stets auf gleiche Art und Weise verformen, insbesondere zeitgleich verformt werden. Ebenso ist es mittels einer zeitversetzten Steuerung möglich, eine peristaltische Förderwirkung mittels der flexiblen Wandungen 8, 8' zu erreichen.

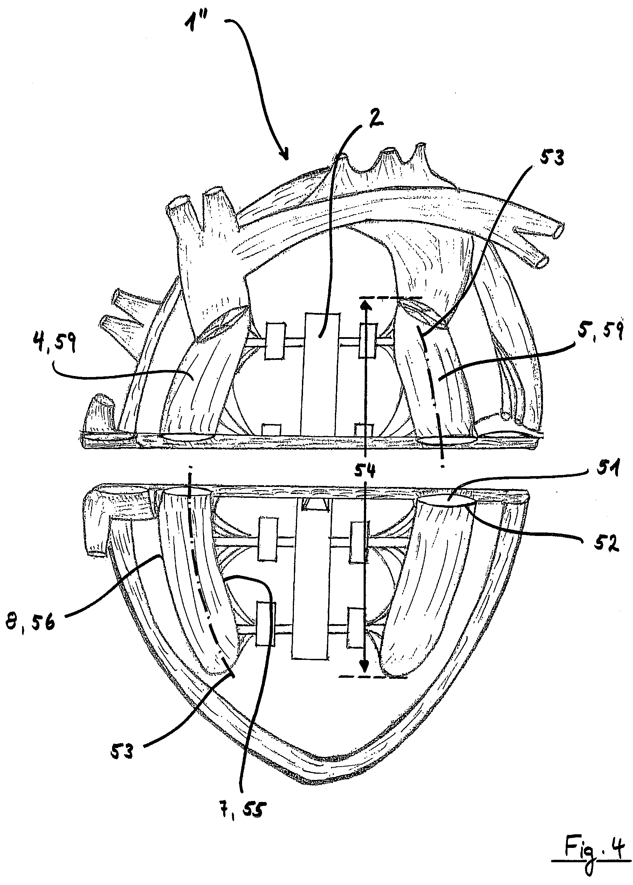

In Figur 4 ist ein Querschnitt durch das vorstehend beschriebene künstliche Herz 1" dargestellt, aus dem die Geometrie der Förderkammern 4,5 deutlich wird. Somit zeigt der Querschnitt deutlich, dass eine Querschnittsfläche 51 der Förderkammern 4, 5 von einer Ellipse 52 eingefasst ist. Die Förderkammern 4, 5 weisen ferner eine hier mittels einer strichpunktierten Linie dargestellte Mittellinie 53 auf, die einen Krümmungsradius aufweist. Die Krümmung der Förderkammern 4, 5 beschreibt im gezeigten Beispiel einen Kreisbogen. Alternativ ist es aber ebenso denkbar, dass ein Krümmungsradius der Mittellinie 53 einer jeweiligen Förderkammer 4, 5 über die Länge derselben veränderlich ist. Im gezeigten Beispiel beträgt der Krümmungsradius der Mittellinie 53 20 cm. Die Mittellinie 53 ist als Verbindungslinie durch die Schwerpunkte aller Querschnitte durch die jeweilige Förderkammer 4, 5 auffassbar.

Die Förderkammern 4, 5 des Herzens 1" weisen eine Länge 54 von ca. 10 cm auf, wobei die Länge 54 einer Förderkammer 4, 5 als Verbindungslinie zwischen den Schwerpunkten von Endquerschnitten der jeweiligen Förderkammer 4, 5 definiert ist, wobei ein die Endquerschnitte einer Förderkammer 4, 5 als diejenigen Querschnitte durch die Förderkammer 4, 5 definiert sind, die senkrecht zur Mittellinie 53 verlaufen und maximal voneinander beabstandet sind.

Eine in Figur 4 nicht dargestellte große Halbachse 57 der Ellipse 52 beträgt 1,8 cm, während die ebenfalls in Figur 4 nicht gezeigte kleine Halbachse 58 1,4 cm beträgt. Das Volumen der Förderkammern 4, 5 berechnet sich demzufolge überschlägig zu ca. V = 80,0 cm³, wobei vereinfacht das Volumen eine Kreisbogens mit elliptischem Querschnitt bestimmt wurde.

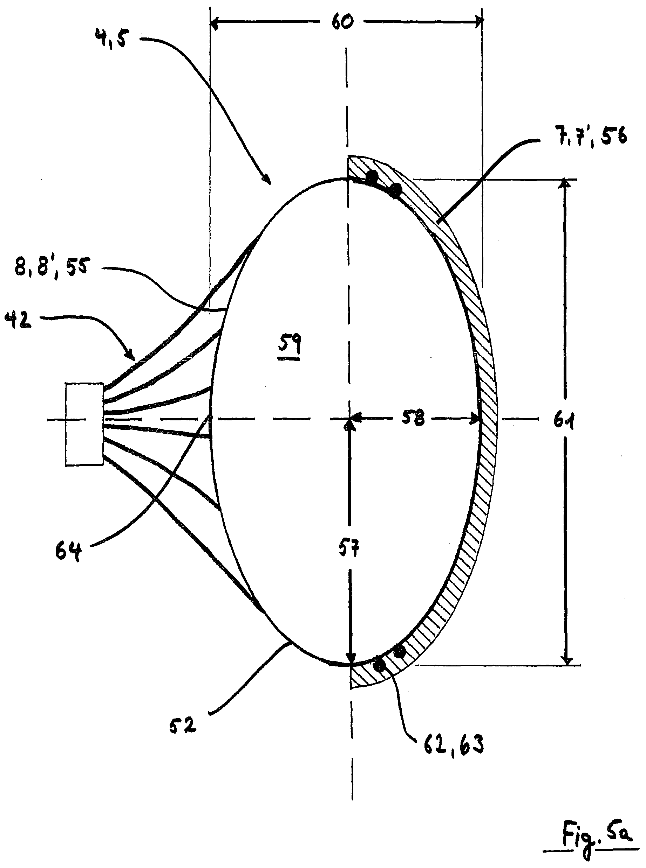

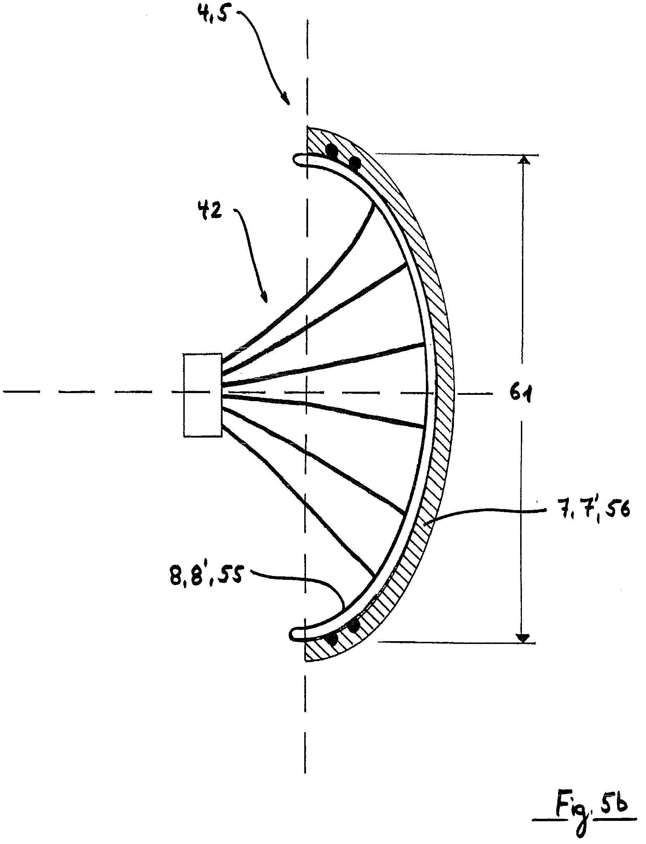

Die Förderkammern 4, 5 weisen jeweils eine elastische Hülle 55 auf, von der die flexiblen Wandungen 8, 8' gebildet sind. Diese elastische Hülle 55 begrenzt einen Innenraum 59 der Förderkammern 4, 5. Ein der Antriebseinrichtung 2 abgewandter Stützbereich der elastischen Hülle 55 der jeweiligen Förderkammern 4, 5 ist mittels eines starren Stützkörpers 56 gekoppelt und dadurch versteift. Der Aufbau der Förderkammern 4, 5 im Hinblick auf die Unterteilung in starre Wandungen 7, 7' und flexible Wandungen 8, 8' ist besonders gut den nachstehend beschriebenen Figuren 5a und 5b entnehmbar.

Diese zeigen einen Querschnitt durch eine der Förderkammern 4, 5 des Herzens 1", wobei die Förderkammer 4, 5 in Figur 5a in ihrer Saugstellung (Volumen maximal) und in Figur 5b in ihrer Druckstellung (Volumen minimal) dargestellt ist. Ein Querschnittsrand weist die Form einer Ellipse 52 auf, wobei die große Halbachse 57 einen Wert von 1,8 cm und die kleine Halbachse 58 einen Wert von 1,4 cm annimmt. Entsprechend beträgt eine Höhe 60 der jeweiligen Förderkammer 4, 5 2,8 cm und eine Breite 61 3,6 cm. Eine Querschnittsfläche des Querschnitts durch die Förderkammer 4, 5 beträgt demzufolge A = π·a·b = 7,9 cm².

Aus Figur 5 wird deutlich, dass der Innenraum 59 der Förderkammer 4, 5 vollständig von der elastischen Hülle 55 eingefasst ist und folglich von diesem begrenzt wird. Der halbe Umfang der elastischen Hülle 55 ist mittels des starren Stützkörpers 56 eingefasst und unter Ausbildung eines Formschlusses mit selbigem in Kraft übertragender Weise verbunden. Der Stützkörper 56 weist hier einen U-förmigen Querschnitt auf, der die elastische Hülle 55 einfasst. Er bildet auf diese Weise einen Stützbereich für die elastische Hülle 55, wodurch diese ausgesteift wird. In Verbindung mit dem Stützkörper 56 ist die elastische Hülle 55 demzufolge nicht mehr flexibel, sondern starr. Entsprechend bildet der Stützbereich der elastischen Hülle 55 die "starre Wandung" 7, 7', während der übrige Bereich, in dem sich die elastische Hülle 55 frei verformen kann, die flexible Wandung 8, 8' der jeweiligen Förderkammer 4, 5 bildet. Der Stützkörper 56 erstreckt sich über eine gesamte Länge der jeweiligen Förderkammer 4, 5.

Der Formschluss zwischen der elastischen Hülle 55 und dem Stützkörper 56 ist mittels eines Formschlusses durch Zusammenwirken von Nut und Feder ausgebildet. Der Stützkörper 56 weist hierzu insgesamt vier Nuten 62 auf, die sich parallel zur Mittellinie 53 der jeweiligen Förderkammer 4, 5 über eine gesamte Länge der Förderkammer 4, 5 erstrecken. Die Nuten 62 sind vorteilhafterweise hinterschnitten ausgeführt. Die elastische Hülle 55 weist mit den Nuten 62 korrespondierende Federn 63 auf, die passgenau in die Nuten 62 eingreifen. Zusätzlich zu dieser Verbindung der elastischen Hülle 55 mit dem Stützkörper 56 kann vorteilhafterweise eine vollflächige Verklebung vorgesehen sein.

Ausgehend von der in Figur 5 dargestellten Saugstellung der flexiblen Wandung 8, 8' muss eine "tiefste Stelle" 64 der flexiblen Wandung 8, 8', das heißt die Stelle des Querschnitts durch die Förderkammer 4, 5, die der Antriebseinrichtung 2 am nächsten ist, um die Höhe 60 der Förderkammer 4, 5 ausgelenkt werden, damit das Volumen der Förderkammer 4, 5 in dessen Druckstellung minimal, vorzugsweise gleich null, ist.

Letztgenannte Druckstellung ist - wie beschrieben - in Figur 5b dargestellt. Hier ist insbesondere besonders gut erkennbar, dass die elastische Hülle 55 vollständig gegen den Stützkörper 56 gedrückt ist, wodurch das Volumen des Innenraums 59 der Förderkammer 4, 5 auf ungefähr null reduziert wird. Das in der Förderkammer 4, 5 befindliche Blut wird demzufolge vollständig verdrängt.

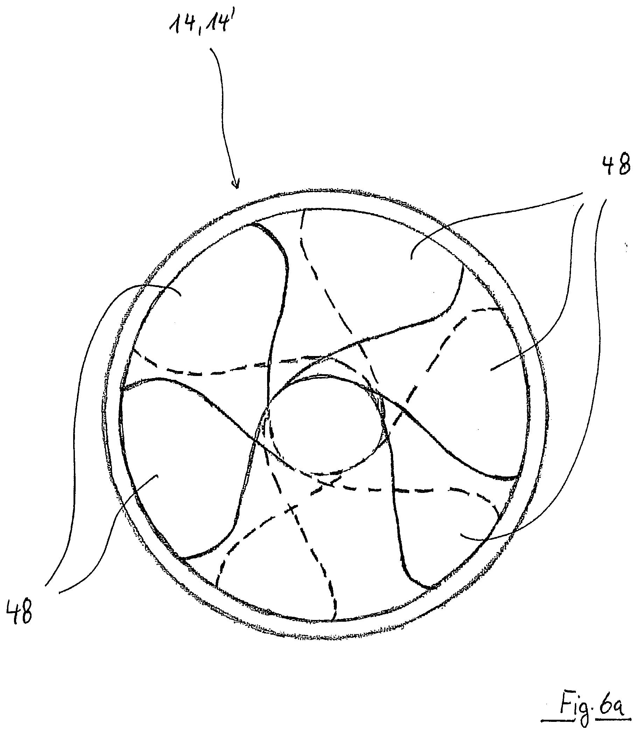

In den Figuren 6a bis 6c sind unterschiedliche Darstellungen eines Klappelements 14, 14' dargestellt, welche die Funktion eines Rückschlagventils übernimmt und jeweils an den Eintrittsöffnungen 9, 10 und den Austrittsöffnungen 11, 12 angeordnet ist.

Figur 6a zeigt eine Draufsicht des in einer Schließstellung befindlichen Klappelements 14 ,14', wobei sich in dieser Schließstellung einzelne Klappsegmente 48 des Klappelements 14, 14' überlappen und auf diese Weise eine Öffnung verschließen. In der gezeigten Konfiguration kann ein Fluidstrom das Klappelemente 14, 14' nicht in eine zur Zeichenebene senkrechte Richtung durchströmen. Die einzelnen Klappsegmente 48 sind dabei entlang eines Öffnungsquerschnitts angeordnet, wobei im gezeigten Beispiel insgesamt fünf Klappsegmente 48 vorhanden sind, die gleichmäßig umlaufend in dem Öffnungsquerschnitt platziert sind.

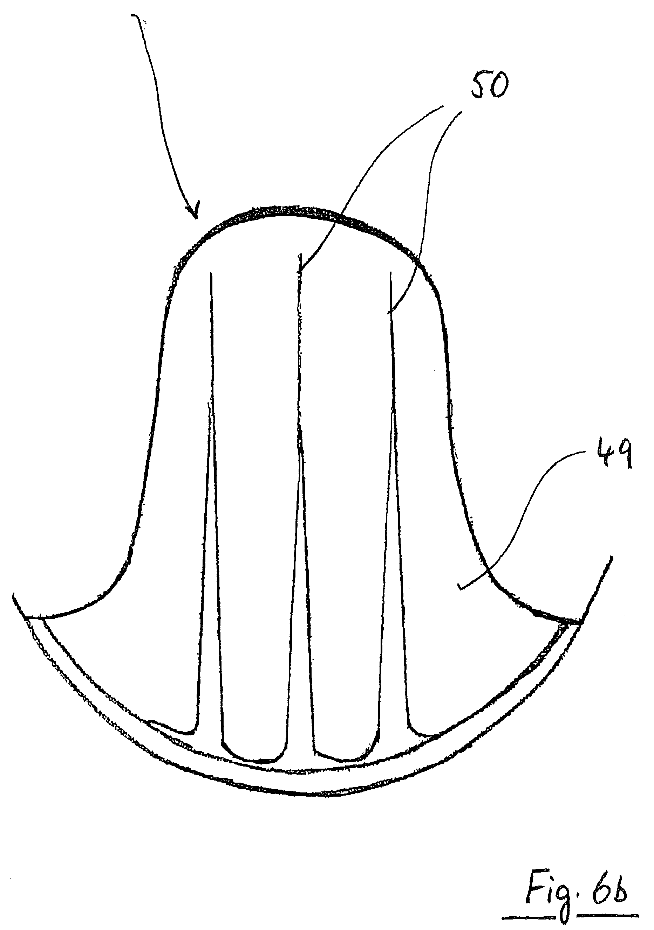

Jedes einzelne Klappsegment 48 hat in etwa eine Form einer Normalparabel, wie aus Figur 6b deutlich wird. Es umfasst ein flächiges Segment-Blatt 49 sowie drei Verstärkungselemente 50, die ein Durchschlagen des Klappsegmentes 48 über die Schließstellung hinaus verhindern. Aufgrund dieser Verstärkungselemente 50 können die Klappsegmente 48 ausschließlich in eine Richtung, in die der Fluidstrom freigebbar sein soll, geöffnet werden.

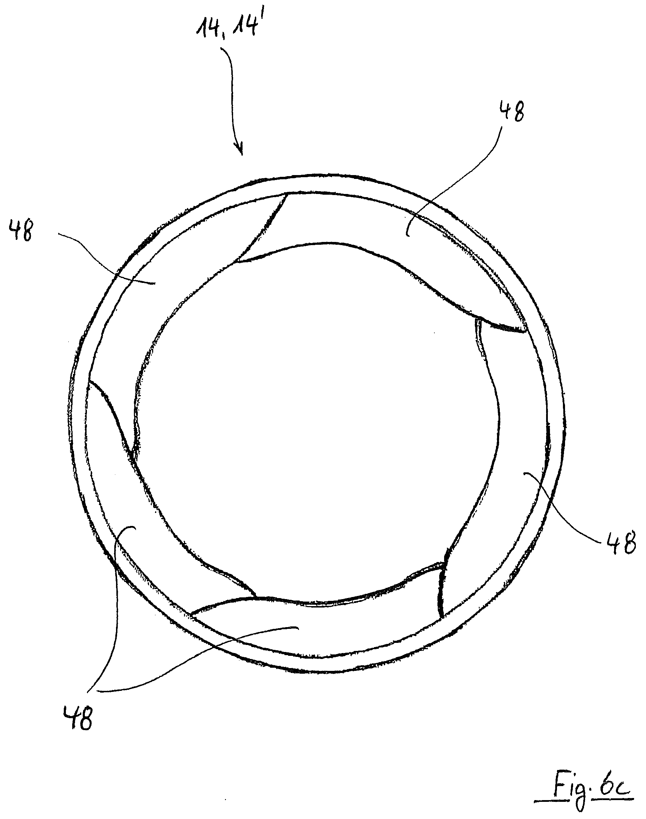

Eine solche Offenstellung des Klappelements 14, 14' ist schließlich aus Figur 6c entnehmbar. Die einzelnen Klappsegmente 48 sind hier "hochgeklappt", so dass sie im Wesentlichen parallel zum Fluidstrom ausgerichtet sind. Die Öffnung ist in dieser Stellung freigegeben. Bei einer Umkehr des Fluidstroms in eine Richtung, in der das Klappelement 14, 14' schließen soll, werden die Klappsegmente 48 automatisch durch eine Bewegung des Fluidstroms aus der Offenstellung in ihre Schließstellung gezwungen, so dass das Funktionsprinzip eines Rückschlagventils nachempfunden ist.

### Bezugszeichenliste

- 1, 1', 1": Herz
- 2: Antriebseinrichtung
- 3: Energiespeicher
- 4: Förderkammer
- 5: Förderkammer
- 6: Hüllkörper
- 7, 7': starre Wandung
- 8, 8': flexible Wandung
- 9: Eintrittsöffnung
- 10: Eintrittsöffnung
- 11: Austrittsöffnung
- 12: Austrittsöffnung
- 13: Wandung
- 14, 14': Klappelement
- 15: Pumpe
- 16: Arbeitsraum
- 17: Arbeitsraum
- 18: Ausgleichraum
- 19: Leitung
- 20: Leitung
- 21: Leitung
- 22: Leitung
- 23: Leitung
- 24: Leitung
- 25: Anschlussraum
- 26: Anschlussraum
- 27, 27': Leitung
- 28, 28', 28", 28'": Leitung
- 29: Antriebsmechanik
- 30: Motor
- 31: Motor
- 32: Schneckenwelle
- 33: Zahnrad
- 34: Zahnrad
- 35: Zahnrad
- 36: Nocken
- 37: Nocken
- 38: Nocken
- 39: Stößel
- 40: Stößel
- 41: Erhebung
- 42: Auffächerung
- 43: Randbereich
- 44: Kolben
- 45: Kolben-Zylinder-Einheit
- 46: Pumpe
- 47: Zylinder
- 48: Klappsegment
- 49: Segment-Blatt
- 50: Verstärkungselement
- 51: Querschnittsfläche
- 52: Ellipse
- 53: Mittellinie
- 54: Länge
- 55: elastische Hülle
- 56: Stützkörper
- 57: große Halbachse
- 58: kleine Halbachse
- 59: Innenraum
- 60: Höhe
- 61: Breite
- 62: Nut
- 63: Feder
- 64: tiefste Stelle

## Patentansprüche

1. Künstliches Herz (1, 1', 1"), insbesondere zur Nachbildung eines menschlichen Herzens, umfassend einen Energiespeicher (3), eine Antriebseinrichtung (2) sowie zwei Förderkammern (4, 5) mit jeweils einer Eintrittsöffnung (9, 10) sowie einer Austrittsöffnung (11, 12), wobei jede Förderkammer (4, 5) mindestens von einer flexiblen Wandung (8, 8') und einer starren Wandung (7, 7') begrenzt ist und ein Volumen jeder Förderkammer (4, 5) durch Verformung der flexiblen Wandung (8, 8') mittels der Antriebseinrichtung (2) veränderbar ist, indem die flexible Wandung (8, 8') ausgehend von einer Saugstellung, in der das Volumen der jeweiligen Förderkammer (4, 5) maximal ist, mittels der Antriebseinrichtung (2) in eine Druckstellung überführbar ist, in der das Volumen der Förderkammer (4, 5) minimal ist, wobei die Eintrittsöffnung (9, 10) und die Austrittsöffnung (11, 12) einer jeden Förderkammer (4, 5) jeweils ein Rückschlagventil aufweisen und die Rückschlagventile wechselseitig in eine geöffnete und eine geschlossene Stellung bringbar sind, wodurch ein gerichteter Blutstrom von der jeweiligen Eintrittsöffnung (9, 10) zu der jeweiligen Austrittsöffnung (11, 12) erzeugbar ist, wobei die Antriebseinrichtung (2) zwischen den Förderkammern (4, 5) angeordnet ist, **dadurch gekennzeichnet, dass** die Förderkammern (4, 5) lang gestreckt und bananenförmig ausgeformt sind, wobei eine Mittellinie (53) einer jeweiligen Förderkammer (4, 5) gekrümmt ist und einen Krümmungsradius zwischen 10 cm und 40 cm aufweist und ein senkrecht zu der Mittellinie (53) einer jeweiligen Förderkammer (4, 5) geführter Querschnitt durch die Förderkammer (4, 5) in der Saugstellung eine Form einer Ellipse (52) aufweist, wobei ein Verhältnis zwischen einer großen Halbachse (57) und einer kleinen Halbachse (58) der Ellipse (52) zwischen 1,0 und 2,0 liegt.

2. Künstliches Herz (1, 1', 1") nach Anspruch 1, **dadurch gekennzeichnet, dass** ein Innenraum (59) einer jeweiligen Förderkammer (4 ,5) von einer elastischen Hülle (55) begrenzt ist, wobei die elastische Hülle (55) in einem von der Antriebseinrichtung (2) abgewandten Stützbereich durch Zusammenwirken mit einem starren Stützkörper (56) die starre Wandung (7, 7') der Förderkammer (4, 5) ausbildet und außerhalb des Stützbereichs die elastische Hülle (55) die flexible Wandung (8, 8') ausbildet.

3. Künstliches Herz (1, 1', 1") nach Anspruch 2, **dadurch gekennzeichnet, dass** die elastische Hülle (55) und der Stützkörper (56) mittels eines Formschlusses in Kraft übertragender Weise miteinander verbunden sind.

4. Künstliches Herz (1, 1', 1") nach einem der Ansprüche 1 bis 3, **gekennzeichnet durch** einen Hüllkörper (6), der beide Förderkammern (4, 5) sowie die Antriebseinrichtung (2) vollständig umschließt.

5. Künstliches Herz (1, 1', 1") nach einem der Ansprüche 1 bis 4, **dadurch gekennzeichnet, dass** bei einer der beiden Förderkammern (4, 5) eine Anordnung der zugehörigen Austrittsöffnung (11, 12) einer natürlichen Anordnung einer Lungenarterie einer rechten Herzkammer des menschlichen Herzens entspricht.

6. Künstliches Herz (1, 1', 1") nach einem der Ansprüche 1 bis 5, **dadurch gekennzeichnet, dass** bei einer der beiden Förderkammern (4, 5) eine Anordnung der zugehörigen Austrittsöffnung (11, 12) einer natürlichen Anordnung einer Aorta einer linken Herzkammer des menschlichen Herzens entspricht.

7. Künstliches Herz (1, 1', 1") nach einem der Ansprüche 1 bis 6, **dadurch gekennzeichnet, dass** bei einer der beiden Förderkammern (4) auf einer der Förderkammer (4) abgewandten Seite der zugehörigen Eintrittsöffnung (9) ein Anschlussraum (25) angeordnet ist.

8. Künstliches Herz (1, 1', 1") nach einem der Ansprüche 1 bis 7, **dadurch gekennzeichnet, dass** bei einer der beiden Förderkammern (5) auf einer der Förderkammer (5) abgewandten Seite der zugehörigen Eintrittsöffnung (10) ein Anschlussraum (26) angeordnet ist.

9. Künstliches Herz (1', 1") nach einem der Ansprüche 1 bis 8, **dadurch gekennzeichnet,**
**dass** mindestens eine flexible Wandung (8, 8') mindestens einer Förderkammer (4, 5) mittels mindestens einer Übertragungseinrichtung bewegt wird, wobei die mindestens eine Übertragungseinrichtung von der Antriebseinrichtung (2) angetrieben wird.

10. Künstliches Herz (1') nach Anspruch 9, Alternative b), **dadurch gekennzeichnet, dass** der Stößel (39. 40) während einer einzigen Umdrehung des Nockens (36, 37, 38) eine Mehrzahl von Hüben ausführt.

11. Künstliches Herz (1') nach Anspruch 9, Alternative b) oder 10, **dadurch gekennzeichnet, dass** der Nocken (36, 37, 38) mit einer Mehrzahl von Stößeln (39, 40) korrespondiert.

12. Künstliches Herz (1) nach einem der Ansprüche 1 bis 8, **dadurch gekennzeichnet, dass** mindestens eine flexible Wandung (8, 8') mindestens einer Förderkammer (4, 5) mittels eines hydraulischen Drucks bewegbar ist, wobei die flexible Wandung (8, 8') mit einem Übertragungsfluid beaufschlagbar ist, das mittels der Antriebseinrichtung (2) mit einem Druck beaufschlagbar ist, wobei die Antriebseinrichtung (2) eine Pumpe (15) aufweist.

13. Künstliches Herz (1, 1', 1") nach einem der Ansprüche 1 bis 12, **dadurch gekennzeichnet, dass** mindestens ein Rückschlagventil aus einem Klappelement (14, 14') gebildet ist.

14. Künstliches Herz (1', 1") nach einem der Ansprüche 1 bis 13, **dadurch gekennzeichnet, dass** mindestens eine der flexiblen Wandungen (8, 8') einzelne Bereiche aufweist, die mittels der Antriebseinrichtung (2) im Zuge einer Überführung der gesamten flexiblen Wandung (8, 8') von der Saugstellung in die Druckstellung zeitversetzt verformbar sind.

15. Künstliches Herz (1, 1', 1") nach einem der Ansprüche 1 bis 14, **dadurch gekennzeichnet, dass** mindestens ein Rückschlagventil mittels eines einen Formschluss ausbildenden Clip-Systems lösbar an der jeweiligen Eintrittsöffnung (9, 10) und/oder Austrittsöffnung (11, 12) angeordnet ist.

## Claims

1. Artificial heart (1, 1', 1"), in particular for imitating a human heart, comprising an energy storage device (2), an actuator device (2) and two supply chambers (4, 5) each having one inlet opening (9, 10) and one outlet opening (11, 12), wherein each supply chamber (4, 5) is a least defined by one flexible wall (8, 8") and one rigid wall (7, 7") and a volume of each supply chamber (4, 5) can be varied by deforming the flexible wall (8, 8') by means of the actuator device (2), in that the flexible wall (8, 8'), starting from a suction position in which the volume of the supply chamber (4, 5) is at a maximum can be transferred by means of the actuator device (2) into a pressure position, in which the volume of the supply chamber (4, 5) is at a minimum, wherein the inlet opening (9, 10) and the outlet opening (11, 12) of each supply chamber (4, 5) each have a non-return valve and the non-return valves can be alternately brought into an open and close position, through which a directed flow of blood from the relevant inlet opening (9, 10) to the relevant outlet opening (11, 12) can be generated, wherein the actuator device (2) is arranged between the supply chambers (4, 5), **characterised in that** the supply chambers (4, 5) are elongated and in the shape of a banana, wherein a midline (53) of each supply chamber (4, 5) is curved with a curvature radius of between 10 cm and 40 cm, and a cross-section taken though the supply chamber (4, 5) perpendicularly to the midline (53) of the relevant supply chamber (4, 5) is in the form of an ellipse (52) in the suction position, wherein the ratio of the semi-major axis (57) to the semi-minor axis (58) of the ellipse (52) is between 1.0 and 2.0.

2. Artificial heart (1, 1', 1") according to claim 1, **characterised in that** at inner space (59) of a relevant supply chamber (4, 5) is defined by an elastic casing (55), wherein the elastic casing (5) in a support area facing away from the actuator device (2), through interaction with a rigid support element (56) forms the rigid wall (7, 7') of the supply chamber (4, 5) and outside the support area the elastic casing (55) forms the flexible wall (8, 8').

3. Artificial heart (1, 1', 1") according to claim 2 **characterised in that** the elastic casing (55) and the support element (56) are connected to each other in a positively locking manner through which forces can be transmitted.

4. Artificial heart (1, 1', 1") according to any one of claims 1 to 3 **characterised by** a casing element (6) which completely surrounds both supply chambers (4, 5) and the actuator device (2).

5. Artificial heart (1, 1', 1") according to any one of claims 1 to 4 **characterised in that** in the case of one of the two supply chambers (4, 5) an arrangement of the associated outlet opening (11, 12) corresponds to a natural arrangement of a pulmonary artery of a right ventricle of the human heart.

6. Artificial heart (1, 1', 1") according to any one of claims 1 to 5 **characterised in that** in the case of one of the two supply chambers (4, 5) an arrangement of the associated outlet opening (11, 13) corresponds to a natural arrangement of an aorta of a left ventricle of a human heart.

7. Artificial heart (1, 1', 1") according to any one of claims 1 to 6, **characterised in that** in the case of one of the two supply chambers (4) a connecting space (25) is arranged on a side of the associated inlet opening (9) facing away from the supply chamber (4).

8. Artificial heart (1, 1', 1") according to any one of claims 1 to 7, **characterised in that** in the case of one of the two supply chambers (5) a connecting space (26) is arranged on a side of the associated inlet opening (10) facing away from the supply chamber (5).

9. Artificial heart (1', 1") according to any one of claims 1 to 8, **characterised in that** at least one flexible wall (8, 8') of at least one supply chamber (4, 5) is moved by means of at least one transmission device, wherein the at least one transmission device is driven by the actuator device (2).

10. Artificial heart (1') according to claim 9, alternative b) **characterised in that** the plunger (39, 40) carries out a plurality of strokes during a single revolution of the cam (36, 37, 38).

11. Artificial heart (1') according to claim 9, alternative b) or 10, **characterised in that** the cam (36, 37, 38) corresponds with a plurality of plungers (39, 40).

12. Artificial heart (1) according to any one of claims 1 to 8, **characterised in that** at least one flexible wall (8, 8') of at least one supply chamber (4, 5) can be moved by means of a hydraulic pressure, wherein a transmission fluid, which can pressurised by means of the actuator device (2), can act on the flexible wall (8, 8'), wherein the actuator device (2) has a pump (15) .

13. Artificial heart (1, 1', 1") according to any one of claims 1 to 12 **characterised in that** at least one non-return valve is formed of a flap element (14, 14').

14. Artificial heart (1', 1") according on any one of claims 1 to 13 **characterised in that** at least one of the flexible walls (8, 8') has individual areas which, during the course of transferring the entire flexible wall (8, 8') from the suction position into the pressure position, can be deformed by means of the actuator unit (2) in a time-offset manner.

15. Artificial heart (1, 1', 1") according to any one of claims 1 to 14 **characterised in that** at least one non-return valve is detachably arranged on the relevant inlet opening (9, 10) and/or outlet opening (11, 12) by means of a clip system forming a positive lock connection.

## Revendications

1. Coeur artificiel (1, 1', 1"), en particulier destiné à reproduire un coeur humain, comprenant un accumulateur d'énergie (3), un dispositif d'entraînement (2) et deux chambres de transport (4, 5) dotées respectivement d'un orifice d'entrée (9, 10) et d'un orifice de sortie (11, 12), chaque chambre de transport (4, 5) étant limitée au moins par une paroi souple (8, 8') et une paroi rigide (7, 7') et un volume de chaque chambre de transport (4, 5) étant modifiable par déformation de la paroi souple (8, 8') au moyen du dispositif d'entraînement (2), du fait que la paroi souple (8, 8'), en partant d'une position d'aspiration dans laquelle le volume de la chambre de transport respective (4, 5) est maximal, (2) peut être amenée au moyen du dispositif d'entraînement (2) dans une position de pression dans laquelle le volume de la chambre de transport (4, 5) est minimal, l'orifice d'entrée (9, 10) et l'orifice de sortie (11, 12) de chaque chambre de transport (4, 5) présentant respectivement une soupape antiretour et les soupapes antiretour pouvant être amenées alternativement dans une position ouverte et une position fermée, ce qui a pour effet qu'un flux sanguin dirigé de l'orifice d'entrée respectif (9, 10) vers l'orifice de sortie respectif (11, 12) peut être généré, le dispositif d'entraînement (2) étant disposé entre les chambres de transport (4, 5), **caractérisé en ce que** les chambres de transport (4, 5) sont étirées en longueur et réalisées en forme de bananes, une ligne médiane (53) d'une chambre de transport respective (4, 5) étant courbée et présentant un rayon de courbure compris entre 10 cm et 40 cm et une section transversale guidée perpendiculairement à la ligne médiane (53) d'une chambre de transport respective (4, 5) dans la chambre de transport (4, 5) présentant, en position d'aspiration, la forme d'une ellipse (52), un rapport entre un grand demi-axe (57) et un petit demi-axe (58) de l'ellipse (52) se situant entre 1,0 et 2,0.

2. Coeur artificiel (1, 1', 1") selon la revendication 1, **caractérisé en ce qu'**un espace intérieur (59) d'une chambre de transport respective (4 ,5) est limité prendre de par une enveloppe élastique, l'enveloppe élastique (55) constituant, dans une zone de soutien détournée du dispositif d'entraînement, par interaction avec un corps de soutien rigide (56) la paroi rigide (7, 7') de la chambre de transport (4, 5) et l'enveloppe élastique (55) constituant la paroi souple (8, 8') à l'extérieur de la zone de soutien.

3. Coeur artificiel (1, 1', 1") selon la revendication 2, **caractérisé en ce que** l'enveloppe élastique (55) et le corps de soutien (56) sont reliés entre eux par correspondance géométrique de manière à transférer la force.

4. Coeur artificiel (1, 1', 1") selon une des revendications 1 à 3, **caractérisée par** un corps enveloppant (6) qui entoure totalement les deux chambres de transport (4, 5), de même que le dispositif d'entraînement (2).

5. Coeur artificiel (1, 1', 1") selon une des revendications 1 à 4, **caractérisé en ce que**, dans une des deux chambres de transport (4, 5), une disposition que l'orifice de sortie correspondant (11, 12) correspond à une disposition naturelle d'une artère pulmonaire d'un ventricule droit du coeur humain.

6. Coeur artificiel (1, 1', 1") selon une des revendications 1 à 5, **caractérisé en ce que**, dans une des deux chambres de transport (4, 5), une disposition que l'orifice de sortie correspondant (11, 12) correspond à une disposition naturelle d'une aorte d'un ventricule gauche du coeur humain.

7. Coeur artificiel (1, 1', 1") selon une des revendications 1 à 6, **caractérisé en ce que**, dans une des deux chambres de transport (4), sur une face détournée de la chambre de transport (4) de l'orifice d'entrée correspondant (9), un espace de raccordement (25) est disposé.

8. Coeur artificiel (1, 1', 1") selon une des revendications 1 à 7, **caractérisé en ce que**, dans une des deux chambres de transport (5), sur une face détournée de la chambre de transport (4) de l'orifice d'entrée correspondant (10), un espace de raccordement (26) est disposé.

9. Coeur artificiel (1, 1") selon une des revendications 1 projet coûts à 8, **caractérisé en ce qu'**au moins une paroi souple (8, 8') d'au moins une chambre de transport (4, 5) est déplacée au moyen d'au moins un dispositif de transfert, l'au moins un dispositif de transfert étant entraîné par le dispositif d'entraînement (2).

10. Coeur artificiel (1') selon la revendication 9, alternative b), **caractérisé en ce que** le poussoir (39, 40) exécute une pluralité de courses pendant une seule rotation de la came (36, 37, 38).

11. Coeur artificiel (1') selon la revendication 9, alternative b) ou 10, **caractérisé en ce que** la came (36, 37, 38) correspond avec une pluralité de poussoirs (39, 40).

12. Coeur artificiel (1) selon une des revendications 1 à 8, **caractérisé en ce qu'**au moins une paroi souple (8, 8') d'au moins une chambre de transport (4, 5) est mobile au moyen d'une pression hydraulique, la paroi souple (8, 8') pouvant être sollicitée par un fluide de transfert qui peut être sollicité au moyen du dispositif d'entraînement (2) par une pression, le dispositif d'entraînement (2) présentant une pompe (15).

13. Coeur artificiel (1, 1', 1") selon une des revendications 1 à 12, **caractérisé en ce qu'**au moins une soupape antiretour est constituée d'un élément rabattable (14, 14').

14. Coeur artificiel (1', 1") selon une des revendications 1 à 13, **caractérisé en ce qu'**au moins une des parois souples (8, 8') présente des zones isolées qui sont déformables avec un décalage chronologique au moyen du dispositif d'entraînement (2) au cours d'un passage de l'ensemble de la paroi souple (8, 8') de la position d'aspiration à la position de pression.

15. Coeur artificiel (1, 1', 1") selon une des revendications 1 à 14, **caractérisé en ce qu'**au moins une soupape antiretour d'au moins un système de clips établissant une correspondance géométrique est disposée de de manière amovible à l'orifice d'entrée (9, 10) et/ou à l'orifice de sortie (11, 12) respectif.
